# EUROPÄISCHE PATENTSCHRIFT

(11) **EP 4 301 335 B1**
(45) Veröffentlichungstag und Bekanntmachung des Hinweises auf die Patenterteilung: **07.05.2025**
(21) Anmeldenummer: 22710997.2
(22) Anmeldetag: 02.03.2022
(51) Int. Cl.: A61K 31/135, A61K 47/32, A61K 9/00

(54) **ORALER DÜNNFILM**
ORAL THIN FILM
FILM ORAL MINCE

(30) Priorität: 04.03.2021 DE 102021105268
(43) Veröffentlichungstag der Anmeldung: 10.01.2024
(73) Patentinhaber: LTS Lohmann Therapie-Systeme AG, 56626 Andernach (DE)
(72) Erfinder: MÜLLER, Markus, 53840 Troisdorf (DE); FICKER, Mario, 53179 Bonn (DE)
(74) Vertreter: Meissner Bolte Partnerschaft mbB
(86) Internationale Anmeldenummer: PCT/EP2022/055284
(87) Internationale Veröffentlichungsnummer: WO 2022/184770

(56) Entgegenhaltungen:
- WO-A1-2014/020155
- DE-A1- 10 328 942
- DE-A1- 102017 129 012

## Beschreibung

Die vorliegende Erfindung betrifft einen oralen Dünnfilm, umfassend mindestens ein Matrixpolymer und mindestens einen pharmazeutisch aktiven Wirkstoff, wobei der mindestens eine pharmazeutisch aktive Wirkstoff eine Säure oder eine Base ist und wobei der mindestens eine pharmazeutisch aktive Wirkstoff in Form einer Mischung vorliegt, die den mindestens einen pharmazeutisch aktiven Wirkstoff sowohl in Form der freien Säure oder Base als auch in Form eines pharmazeutisch akzeptablen Salzes umfasst, ein Verfahren zu dessen Herstellung, sowie die Verwendung eines solchen oralen Dünnfilms als Arzneimittel.

Orale Dünnfilme sind dünne, mindestens einen pharmazeutisch aktiven Wirkstoff enthaltende Filme, die direkt in den Mundraum gelegt oder an die Mundschleimhaut angelegt werden und sich dort auflösen bzw. aufquellen und dabei den Wirkstoff abgeben. Dabei handelt es sich insbesondere um dünne, ein- oder mehrschichtige, wirkstoffhaltige Filme auf Polymerbasis, die, wenn sie auf die Schleimhaut, insbesondere die Mundschleimhaut, aufgebracht werden, den Wirkstoff direkt in diese abgeben können. Die sehr gute Durchblutung der Mundschleimhaut sorgt für einen schnellen Übergang des Wirkstoffs in den Blutkreislauf. Dieses Darreichungssystem hat den Vorteil, dass der Wirkstoff größtenteils durch die Schleimhaut resorbiert wird und damit der "First-Pass Metabolismus", der bei der konventionellen Darreichungsform eines Wirkstoffs in Tablettenform auftritt, vermieden wird. Der Wirkstoff kann in dem Film gelöst, emulgiert oder dispergiert werden.

Für die Resorption eines pharmazeutisch aktiven Wirkstoffs über eine Schleimhaut sowie für die Stabilität eines pharmazeutisch aktiven Wirkstoffes und die Stabilität einer pharmazeutischen Zusammensetzung insgesamt ist der pH-Wert ein entscheidender Faktor. Insbesondere beim Auflösen eines oralen Dünnfilms im Mund eines Patienten ist der resultierende pH-Wert an der Stelle, an der sich der orale Dünnfilm auflöst, entscheidend für die Resorption des pharmazeutisch aktiven Wirkstoffes.

Gewünschte pH-Werte werden üblicherweise durch Zugabe von Säuren oder Basen oder durch Puffersysteme eingestellt, was sich jedoch negativ auf die pharmazeutische Formulierung auswirken kann. Durch die Neutralisation eines Wirkstoffs durch Zugabe von Säure oder Base zu der Formulierung entsteht als Nebenprodukt ein Salz, das die Formulierung negativ beeinflusst, da diesem keine weitere Funktion zukommt. Das entstehende Salz erhöht insbesondere auch das Flächengewicht eines oralen Dünnfilms und kann zudem die Stabilität der Formulierung und den Geschmack negativ beeinflussen. Analoges gilt für den Einsatz von Puffersystemen.

DE 10 2017 129012 offenbart einen oralen Dünnfilm umfassend mindestens ein Cellulosederivat und mindestens einen pharmazeutisch aktiven Wirkstoff, wobei der mindestens eine pharmazeutisch aktive Wirkstoff eine Wasserlöslichkeit von höchstens 50 g/L bei 20°C und einem pH von 6 bis 7 aufweist und in einer Menge von mindestens 20 Gew.-%, bezogen auf das Gesamtgewicht des oralen Dünnfilms, in dem oralen Dünnfilm enthalten ist.

WO 2014/020155 offenbart Verfahren und Zusammensetzungen zur Behandlung von Schmerzen.

DE 103 28 942 offenbart filmförmige Darreichungsformen zur transmukosalen Verabreichung von Wirkstoffen an den menschlichen oder tierischen Körper, die durch eine Anpassung oder Annäherung des pH-Wertes der für die Herstellung der Darreichungsform vorgesehenen Basismasse, umfassend ein Lösungsmittel oder Lösungsmittelgemisch, mindestens ein matrixbildendes Polymer und mindestens einen Wirkstoff, an den physiologischen pH-Wert der für die Applikation vorgesehenen Schleimhaut gekennzeichnet sind.

Die Aufgabe der vorliegenden Erfindung besteht darin, vorstehend genannte Nachteile des Standes der Technik zu beheben. Insbesondere besteht die Aufgabe der vorliegenden Erfindung darin, einen oralen Dünnfilm bereitzustellen, dessen pH-Wert, insbesondere der pH-Wert nach der Auflösung des oralen Dünnfilms, vorteilhaft eingestellt werden kann, ohne dass dem oralen Dünnfilm weitere Säure, Base oder Puffersysteme zugesetzt werden müssen. Ferner soll durch den oralen Dünnfilm die Einstellung des pH-Wertes im Speichel des Patienten in einem möglichst großen Bereich möglich und dabei möglichst stabil sein. Zudem soll der orale Dünnfilm möglichst einfach und wirtschaftlich herstellbar sein.

Obige Aufgabe wird durch einen mehrschichtigen oralen Dünnfilm nach Anspruch 1 gelöst, insbesondere durch einen oraler Dünnfilm, umfassend mindestens ein Matrixpolymer und mindestens einen pharmazeutisch aktiven Wirkstoff, wobei der mindestens eine pharmazeutisch aktive Wirkstoff eine Säure oder eine Base ist, der dadurch gekennzeichnet ist, dass der mindestens eine pharmazeutisch aktive Wirkstoff in Form einer Mischung vorliegt, die den mindestens einen pharmazeutisch aktiven Wirkstoff sowohl in Form der freien Säure oder Base als auch in Form eines pharmazeutisch akzeptablen Salzes umfasst, wobei die Mischung, die den mindestens einen pharmazeutisch aktiven Wirkstoff sowohl in Form der freien Säure oder Base als auch in Form eines pharmazeutisch akzeptablen Salzes umfasst, den mindestens einen pharmazeutisch aktiven Wirkstoff in Form der freien Säure oder Base und den mindestens einen pharmazeutisch aktiven Wirkstoff in Form eines pharmazeutisch akzeptablen Salzes der freien Säure oder Base im molaren Verhältnis von 3:1 bis 1:3 umfasst.

Ein solcher oraler Dünnfilm ist vorteilhaft, da durch die Mischung, die den mindestens einen pharmazeutisch aktiven Wirkstoff sowohl in Form der freien Säure oder Base als auch in Form eines pharmazeutisch akzeptablen Salzes umfasst, ein Puffersystem in dem oralen Dünnfilm bereitgestellt wird, ohne dass weitere Säuren, Basen oder Puffersubstanzen zugegeben werden müssen. Durch Einstellung des Verhältnisses des mindestens einen pharmazeutisch aktiven Wirkstoffs in Form der freien Säure oder Base und des mindestens einen pharmazeutisch aktiven Wirkstoffs in Form eines pharmazeutisch akzeptablen Salzes kann der pH-Wert der Formulierung in eingestellt werden. Dadurch, dass einer solchen Formulierung keine weitere Säure, Base oder Puffersubstanz zugegeben werden muss, wird die Formulierung und damit der finale orale Dünnfilm auch nicht durch das Entstehen von Salzen während der Neutralisation oder das Vorliegen eines Puffersystems negativ beeinflusst.

Zur Definition von Säure und Base ist insbesondere das dem Fachmann bekannte Brønsted Säure-Base-Konzept heranzuziehen. Damit umfasst der erfindungsgemäße orale Dünnfilm insbesondere mindestens einen pharmazeutisch aktiven Wirkstoff, der gemäß des Brønsted Säure-Base-Konzeptes eine Säure oder eine Base ist.

In der vorliegenden Schrift kann "umfassend" auch "bestehend aus" bedeuten.

Der erfindungsgemäße orale Dünnfilm ist ferner vorzugsweise dadurch gekennzeichnet, dass das mindestens eine Matrixpolymer ein wasserlösliches Polymer umfasst.

Wasserlösliche Polymere umfassen chemisch sehr unterschiedliche, natürliche oder synthetische Polymere, deren gemeinsames Merkmal ihre Löslichkeit in Wasser oder wässrigen Medien ist. Voraussetzung ist, dass diese Polymere eine für die Wasserlöslichkeit ausreichende Anzahl an hydrophilen Gruppen besitzen und nicht vernetzt sind. Die hydrophilen Gruppen können nicht-ionisch, anionisch, kationisch und/oder zwitterionisch sein.

Unter wasserlöslich wird vorzugsweise eine Löslichkeit von größer als 100 g/L in Wasser bei 25°C verstanden.

Das mindestens eine wasserlösliche Polymer ist vorzugsweise ausgewählt aus der Gruppe umfassend Stärke und Stärkederivate, Dextrane, Cellulosederivate, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylate, Polyvinylpyrrolidone, Vinvlpyrrolidon/Vinylacetat-Copolymere, Polyvinylalkohole, Polyethylenoxidpolymere, Polyacrylamide, Polyethylenglycole, Gelatine, Collagen, Alginaten, Pektin, Pullulan, Traganth, Chitosan, Alginsäure, Arabinogalaktan, Galaktomannan, Agar, Agarose, Carrageen und natürlichen Gummen, Polyvinylalkohol-Polyethylenglycol-Pfropfcopolymere, wobei Polyvinylalkohole besonders bevorzugt sind.

Der erfindungsgemäße orale Dünnfilm ist ferner vorzugsweise dadurch gekennzeichnet, dass das mindestens eine Matrixpolymer, vorzugsweise das wasserlösliche Polymer, in einer Menge von 10 bis 90 Gew.-%, vorzugsweise von 20 bis 60 Gew.-%, besonders bevorzugt von 30 bis 50 Gew.-%, bezogen auf das Gesamtgewicht des oralen Dünnfilms, vorliegt.

Der erfindungsgemäße orale Dünnfilm ist vorzugsweise dadurch gekennzeichnet, dass der orale Dünnfilm neben der Mischung, die den mindestens einen pharmazeutisch aktiven Wirkstoff sowohl in Form der freien Säure oder Base als auch in Form eines pharmazeutisch akzeptablen Salzes umfasst, keine weiteren Säure Basen, Salze und/oder Puffersysteme umfasst.

Insbesondere soll der orale Dünnfilm keine Salzsäure, kein NaOH, kein KOH kein Na₂CO₃, kein NaHCO₃, kein K₂CO₃ und/oder keine Schwefelsäure enthalten.

Ferner soll der erfindungsgemäße orale Dünnfilm kein(en) NaCl, KCl, Phosphatpuffer, TRIS-Puffer, Citratpuffer, Carbonatpuffer, Sulfatpuffer, Boratpuffer und/oder Amoniumpuffer enthalten.

Der mindestens eine pharmazeutisch aktive Wirkstoff unterliegt abgesehen davon, dass er eine Säure oder eine Base ist, prinzipiell keiner Beschränkung, ist aber vorzugsweise ausgewählt aus allen pharmazeutisch aktiven Wirkstoffen, die zur oralen und/oder transmucosalen Applikation geeignet sind.

Vorzugsweise ist der erfindungsgemäße orale Dünnfilm, dadurch gekennzeichnet, dass der mindestens eine pharmazeutisch aktive Wirkstoff, wenn er eine Säure ist, einen pKs von 3 bis 11, vorzugsweise von 4 bis 9 aufweist.

Vorzugsweise ist der erfindungsgemäße orale Dünnfilm, dadurch gekennzeichnet, dass der mindestens eine pharmazeutisch aktive Wirkstoff, wenn er eine Base ist, einen pK_{b} von 3 bis 11, vorzugsweise von 4 bis 9 aufweist.

Vorzugsweise ist der erfindungsgemäße orale Dünnfilm, dadurch gekennzeichnet, dass der mindestens eine pharmazeutisch aktive Wirkstoff eine Carboxylgruppe, eine Aminogruppe, eine Sulfongruppe und/oder eine Phosphonatgruppe umfasst.

Bevorzugt sind Wirkstoffe ausgewählt aus der Gruppe, umfassend die Wirkstoffklassen der Analgetika, Hormone, Hypnotika, Sedativa, Antiepileptika, Weckamine, Psychoneurotropika, Neuro-Muskelblocker, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormone, Sexualhormone, Antidiabetika, Antitumor-Wirkstoffen, Antibiotika, Chemotherapeutika und Narcotika, wobei diese Gruppe nicht abschließend ist.

Besonders bevorzugt handelt es sich bei dem mindestens einen pharmazeutisch aktiven Wirkstoff um Ketamin.

Unter Ketamin wird (S)-(±)-2-(2-Chlorphenyl)-2-(methylamino)cyclohexan-1-on, (R)-(±)-2-(2-Chlorphenyl)-2-(methylamino)cyclohexan-1-on, sowie das Racemat (RS)-(±)-2-(2-Chlorphenyl)-2-(methylamino)cyclohexan-1-on verstanden.

Besonders bevorzugt liegt (S)-Ketamin als einziges Stereoisomer von Ketamin vor, da die analgetische und anästhetische Potenz von (S)-Ketamin etwa dreifach höher als die der (R)-Form ist.

Vorzugsweise ist der erfindungsgemäße orale Dünnfilm, dadurch gekennzeichnet, dass die Mischung, die den mindestens einen pharmazeutisch aktiven Wirkstoff sowohl in Form der freien Säure oder Base als auch in Form eines pharmazeutisch akzeptablen Salzes umfasst, Ketamin als freie Base und Ketaminhydrochlorid, bevorzugt (S)-Ketamin als freie Base und (S)-Ketaminhydrochlorid, umfasst.

Durch die Mischung von Ketamin als freie Base und Ketaminhydrochlorid können insbesondere geeignete pH-Werte eingestellt werden, ohne dass weitere Säuren, Basen oder Puffersysteme zugegeben werden müssen.

Der Wirkstoffgehalt in dem oralen Dünnfilm kann innerhalb relativ weiten Grenzen variieren. Als geeignet kann ein Bereich von 10 bis 60 Gew.-%, bezogen auf das Gesamtgewicht (Trockengesamtgewicht) des oralen Dünnfilms, angegeben werden. In einer Ausführungsform liegt der Anteil an Wirkstoff in dem oralen Dünnfilm eher im unteren Bereich, z.B. wenn der Wirkstoff einen stark unangenehmen Geschmack aufweist, der mit einer größeren Menge an geschmacksmaskierenden Mitteln kompensiert werden muss. In diesem Fall kann als geeigneter Wirkstoffanteil ein Bereich von 10 bis 40 Gew.-% angegeben werden. In einer anderen Ausführungsform liegt der Anteil an Wirkstoff in der erfindungsgemäßen Darreichungsform eher im oberen Bereich, wobei ein Gehalt von 40 bis 60 Gew.-% und insbesondere ein Gehalt von 45 bis 55 Gew.-% als besonders bevorzugt angegeben werden kann.

Die Menge an pharmazeutisch aktivem Wirkstoff bezieht sich dabei auf die Mischung, die den mindestens einen pharmazeutisch aktiven Wirkstoff sowohl in Form der freien Säure oder Base als auch in Form eines pharmazeutisch akzeptablen Salzes umfasst, also auf die Summe der Mengen des mindestens einen pharmazeutisch aktiven Wirkstoffs in Form der freien Säure oder Base und die Menge des mindestens einen pharmazeutisch aktiven Wirkstoffs in Form eines pharmazeutisch akzeptablen Salzes davon.

Besonders bevorzugt ist der erfindungsgemäße orale Dünnfilm daher dadurch gekennzeichnet, dass die die Mischung, die den mindestens einen pharmazeutisch aktiven Wirkstoff sowohl in Form der freien Säure oder Base als auch in Form eines pharmazeutisch akzeptablen Salzes umfasst, in einer Menge von 35 bis 60 Gew.-%, bezogen auf das Gesamtgewicht des oralen Dünnfilms, vorliegt.

Besonders bevorzugt liegt Ketamin als freie Base und als ein pharmazeutisch akzeptables Salz davon, insbesondere Ketamin als freie Base und als Ketaminhydrochlorid, bevorzugt (S)-Ketamin als freie Base und als (S)-Ketaminhydrochlorid, in Summe in einer Menge von 35 bis 60 Gew.-%, bezogen auf das Gesamtgewicht des oralen Dünnfilms, vor.

Zur Einstellung eines pH-Wertes ist das molare Verhältnis des mindestens einen pharmazeutisch aktiven Wirkstoffs in Form der freien Säure oder Base und des mindestens einen pharmazeutisch aktiven Wirkstoffs in Form eines pharmazeutisch akzeptablen Salzes davon ausschlaggebend.

Bevorzugt ist der erfindungsgemäße orale Dünnfilm daher dadurch gekennzeichnet, dass die Mischung, die den mindestens einen pharmazeutisch aktiven Wirkstoff sowohl in Form der freien Säure oder Base als auch in Form eines pharmazeutisch akzeptablen Salzes umfasst, den mindestens einen pharmazeutisch aktiven Wirkstoff in Form der freien Säure oder Base und den mindestens einen pharmazeutisch aktiven Wirkstoff in Form eines pharmazeutisch akzeptablen Salzes im molaren Verhältnis von 1,5 zu 1 bis 1:1,5, bevorzugt etwa 1:1 und besonders bevorzugt 1:1, umfasst.

Bevorzugt beträgt das molare Verhältnis von Ketamin als freie Base und Ketamin als pharmazeutisch akzeptables Salz 3:1 bis 1:3, bevorzugt 1,5 zu 1 bis 1:1,5, besonders bevorzugt etwa 1:1 und ganz besonders bevorzugt 1:1.

Ganz besonders bevorzugt umfasst der erfindungsgemäße orale Dünnfilm Ketamin als freie Base und Ketaminhydrochlorid im molaren Verhältnis von 3:1 bis 1:3, bevorzugt 1,5 zu 1 bis 1:1,5, besonders bevorzugt etwa 1:1 und ganz besonders bevorzugt 1:1.

In einer anderen bevorzugten Ausführungsform umfasst der erfindungsgemäße orale Dünnfilm (S)-Ketamin als freie Base und (S)-Ketaminhydrochlorid im molaren Verhältnis von 3:1 bis 1:3, bevorzugt 1,5 zu 1 bis 1:1,5, besonders bevorzugt etwa 1:1 und ganz besonders bevorzugt 1:1.

Bevorzugt ist der erfindungsgemäße orale Dünnfilm ferner dadurch gekennzeichnet, dass der orale Dünnfilm einen pH von 3,5 bis 9,5, vorzugsweise von 4,5 bis 8,8 aufweist.

Darunter wird der pH-Wert (bei 20°C) verstanden, der sich in einer Lösung einstellt, zu deren Herstellung der orale Dünnfilm in reinem Wasser aufgelöst wird.

Der erfindungsgemäße mehrschichtige orale Dünnfilm ist ferner vorzugsweise dadurch gekennzeichnet, dass die Matrixschicht mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, pH-Regulatoren, Feuchthaltemittel, Konservierungsmittel und/oder Antioxidationsmittel, umfasst.

Jeder dieser Hilfsstoffe ist vorzugsweise jeweils in einer Menge von 0,1 bis 40 Gew.-%, vorzugsweise von 0,1 bis 30 Gew.-%, besonders bevorzugt von 0,1 bis 15 Gew.-%, ganz besonders bevorzugt von 0,1 bis 10 Gew.-% oder 0,1 bis 5 Gew.-%, bezogen auf das Gesamtgewicht der Matrixschicht, in dieser Schicht enthalten.

Der erfindungsgemäße mehrschichtige orale Dünnfilm ist prinzipiell in der Anzahl der enthaltenen Schichten nicht beschränkt.

So sind Ausführungsformen denkbar, bei denen der erfindungsgemäße orale Dünnfilm mehrere wirkstoffhaltige Schichten aufweist.

Der erfindungsgemäße orale Dünnfilm kann in einer Ausführungsform einen im Wesentlichen glatten Film darstellen.

Vorzugsweise ist der erfindungsgemäße orale Dünnfilm dadurch gekennzeichnet, dass er in Form eines Hohlräume aufweisenden verfestigten Schaums vorliegt.

Durch die Hohlräume und die damit verbundene größere Oberfläche der Filme wird insbesondere der Zutritt von Wasser bzw. Speichel oder anderen Körperflüssigkeiten in das Innere der Darreichungsform erleichtert und somit die Auflösung der Darreichungsform und die Wirkstofffreisetzung beschleunigt.

Bei einem schnell resorbierenden Wirkstoff kann zudem durch die schnelle Auflösung der Matrixschicht die transmucosale Resorption verbessert werden.

Zum anderen ist die Wandstärke der genannten Hohlräume vorzugsweise gering, da diese beispielsweise verfestigte Blasen darstellen, so dass eine schnelle Auflösung oder Zerstörung dieser Hohlräume stattfindet.

Ein weiterer Vorteil dieser Ausführungsform besteht darin, dass sich trotz des vergleichsweise hohen Flächengewichts durch die Konfektionierung als Schaum eine schnellere Trocknung realisieren lässt als bei einer vergleichbaren nicht geschäumten Zusammensetzung.

Vorzugsweise ist der erfindungsgemäße orale Dünnfilm dadurch gekennzeichnet, dass die Hohlräume voneinander isoliert sind, und vorzugsweise in Gestalt von Blasen vorliegen, wobei die Hohlräume mit Luft oder einem Gas, vorzugsweise mit einem inerten Gas, besonders bevorzugt mit Stickstoff, Kohlendioxid, Helium oder einem Gemisch mindestens zweier dieser Gase, gefüllt sind.

Gemäß einer anderen Ausführungsform ist vorgesehen, dass die Hohlräume miteinander in Verbindung stehen, vorzugsweise indem sie ein zusammenhängendes, die Matrix durchdringendes Kanalsystem bilden.

Vorzugsweise haben die genannten Hohlräume einen Volumenanteil von 5 bis 98 %, bevorzugt von 40 bis 80 %, bezogen auf das Gesamtvolumen der Matrixschicht. Auf diese Weise wird der vorteilhafte Effekt, die Lösung des oralen Dünnfilms zu beschleunigen, in günstiger Weise beeinflusst.

Ferner können dem oralen Dünnfilm zur Schaumbildung oder dem erhaltenen Schaum vor oder nach dem Trocknen oberflächenaktive Stoffe bzw. Tenside hinzugefügt werden, um die Stabilität des Schaums vor oder nach dem Trocknen zu verbessern.

Ein weiterer Parameter, der die Eigenschaften des erfindungsgemäßen orale Dünnfilms beeinflusst, ist der Durchmesser der Hohlräume oder Blasen. Die Blasen oder Hohlräume werden vorzugsweise mit Hilfe einer Schaumaufschlagmaschine erzeugt, mit der der Durchmesser der Blasen in einem weiten Bereich, fast beliebig, eingestellt werden kann. So kann der Durchmesser der Blasen oder Hohlräume im Bereich von 0,01 bis 60 µm liegen. Besonders bevorzugt liegt der Durchmesser im Bereich von 10 und 50 µm.

Der erfindungsgemäße orale Dünnfilm besitzt vorzugsweise eine Fläche von 0,5 cm² bis 10 cm², besonders bevorzugt von 1,5 cm² bis 8 cm².

Das Flächengewicht des erfindungsgemäßen oralen Dünnfilms beträgt vorzugsweise mindestens 10 g/m², bevorzugter mindestens 20 g/m² oder mindestens 30 g/m² oder am meisten bevorzugt mindestens 50 g/m² oder ist kleiner als oder gleich 400 g/m², bevorzugter kleiner als oder gleich 350 g/m² oder kleiner als oder gleich 300 g/m² oder am meisten bevorzugt kleiner als oder gleich 150 g/m². Vorzugsweise beträgt das Flächengewicht 10 bis 400 g/m², bevorzugter 20 bis 350 g/m² oder 30 bis 300 g/m² und am meisten bevorzugt 50 bis 200 g/m².

Bevorzugt weist der erfindungsgemäße orale Dünnfilm eine Dicke von etwa 10 µm bis etwa 500 µm, besonders bevorzugt von etwa 20 µm bis etwa 300 µm, auf.

Die vorliegende Erfindung betrifft ferner ein Verfahren zur Herstellung des erfindungsgemäßen oralen Dünnfilms umfassend die Schritte:
a) Herstellen einer Lösung, Dispersion oder Schmelze, die mindestens das mindestens eine Matrixpolymer und den mindestens einen pharmazeutischen Wirkstoff in Form einer Mischung, die den mindestens einen pharmazeutisch aktiven Wirkstoff sowohl in Form der freien Säure oder Base als auch in Form eines pharmazeutisch akzeptablen Salzes umfasst, wobei die Mischung, die den mindestens einen pharmazeutisch aktiven Wirkstoff sowohl in Form der freien Säure oder Base als auch in Form eines pharmazeutisch akzeptablen Salzes umfasst, den mindestens einen pharmazeutisch aktiven Wirkstoff in Form der freien Säure oder Base und den mindestens einen pharmazeutisch aktiven Wirkstoff in Form eines pharmazeutisch akzeptablen Salzes der freien Säure oder Base im molaren Verhältnis von 3:1 bis 1:3 umfasst, enthält,
b) Ausstreichen der Lösung, Dispersion oder Schmelze aus Schritt a), um einen Film zu erhalten, und
c) Trocknen des Films aus Schritt b), um einen oralen Dünnfilm zu erhalten.

Das erfindungsgemäße Verfahren umfasst vorzugsweise auch keinen Schritt, bei dem der mindestens eine pharmazeutisch aktive Wirkstoff durch Zugabe von Säure oder Base ganz oder teilweise neutralisiert wird.

Das erfindungsgemäße Verfahren ist vorzugsweise auch dadurch gekennzeichnet, dass kein weiteres Puffersystem in dem oralen Dünnfilm vorhanden ist.

Das erfindungsgemäße Verfahren umfasst vorzugsweise einen optionalen Schritt a1), der das Aufschäumen der Lösung, Dispersion oder Schmelze aus Schritt a) durch Eintragen eines Gases oder Gasgemisches, durch chemische Gaserzeugung oder durch Entspannen eines gelösten Gases umfasst. Dem Fachmann ist klar, dass der Schritt a1) nur dann notwendig ist, wenn der orale Dünnfilm in Form eines Hohlräume aufweisenden verfestigten Schaums vorliegen soll.

Außerdem betrifft die vorliegende Erfindung einen oralen Dünnfilm, wie vorstehend beschrieben, oder erhältlich nach dem vorstehend beschriebenen Verfahren als Arzneimittel.

Die vorliegende Erfindung betrifft zudem einen oralen Dünnfilm, wie vorstehend beschrieben oder erhältlich nach dem vorstehend beschriebenen Verfahren, wobei eine Mischung von Ketamin als freie Base und Ketamin als pharmazeutisch akzeptables Salz, vorzugsweise Ketamin als freie Base und Ketaminhydrochlorid, insbesondere (S)-Ketamin als freie Base und (S)-Ketamin als pharmazeutisch akzeptables Salz, besonders bevorzugt (S)-Ketamin als freie Base und (S)-Ketaminhydrochlorid, eingesetzt wird, zur Verwendung in der Behandlung von Schmerzen und/oder Depressionen, insbesondere zur Reduzierung des Suizidrisikos, und/oder zur Verwendung als Allgemeinanästhetikum, vorzugsweise zur Einleitung und Durchführung einer Vollnarkose oder als Ergänzung bei Regionalanästhesien und/oder als Analgetikum.

Die vorstehend für den erfindungsgemäßen mehrschichtigen oralen Dünnfilm aufgeführten bevorzugten Ausführungsformen gelten auch für das erfindungsgemäße Verfahren, den durch dieses Verfahren erhaltenen mehrschichtigen oralen Dünnfilm und dessen Verwendung als Arzneimittel.

Die Erfindung wird nachfolgend anhand von nicht beschränkenden Beispielen näher erläutert.

### Beispiele

Bei der Entwicklung von oralen Dünnfilm enthaltend Ketamin als pharmazeutisch aktivem Wirkstoff wurde festgestellt, dass der pH-Wert im Mundraum des Patienten einen Einfluss auf die Wirkstofffreisetzung haben kann. Um das eingesetzte Ketaminhydrochlorid, welches in der getesteten Formulierung (siehe Tabelle 1) einen pH von 5,32 aufweist, in einen teilneutralisierten Zustand zu bringen, wurde dieses mit NaOH umgesetzt. Dies führte zu der Bildung von NaCl, welches die Formulierung beeinträchtigt, da es in der Zusammensetzung berücksichtigt werden muss. Bei der Formulierung einer Mischung aus Ketamin als freie Base und Ketaminhydrochlorid im molaren Verhältnis von 1:1 konnte im Wesentlichen der gleiche pH-Wert wie bei einer äquimolaren 50%igen Neutralisation mit NaOH erreicht werden.

**Tabelle 1**

| | | Formulierung [Gew.-%] | | | |
|---|---|---|---|---|---|
| Inhaltsstoff | Funktion | 1 | 2 | 3 | 4 |
| (S)-Ketamin HCl | Wirkstoff | 50,00 | 50,00 | --- | 25,00 |
| (S)-Ketamin Base | Wirkstoff | --- | --- | 43,35 | 21,68 |
| Polyvinylalkohol | Matrixpolymer | 39,10 | 35,85 | 46,15 | 42,82 |
| NaOH | pH-Regulator | --- | 3,65 | --- | --- |
| Saccarin Na | Geschmackskorrigenz | 1,00 | 1,00 | 1,00 | 1,00 |
| Sucrlaose | Geschmackskorrigenz | 2,00 | 2,00 | 2,00 | 2,00 |
| Cherry Flavor | Geschmackskorrigenz | 3,00 | 3,00 | 3,00 | 3,00 |
| Glycerol | Feuchthaltemittel | 4,50 | 2,71 | 4,50 | 4,50 |
| FD&C red | Farbstoff | 0,40 | --- | --- | --- |
| | | | | | |
| pH-Wert | Gemessen als aufgelöster OTF in auf 32 °C temperiertes entmaterialisiertes Wasser (Blindwert pH 7,12) | 5,32 | 6,15 | 7,97 | 6,11 |
| pH-Wert | Gemessen als aufgelöster OTF in auf 32 °C | 5,49 | 6,43 | 7,32 | 6,50 |
| | temperierter Humanspeichel (Blindwert pH 6,95) | | | | |

Orale Dünnfilme der Zusammensetzung gemäß Tabelle 1 wurden folgendermaßen hergestellt. Der Wirkstoff bzw. die Wirkstoffmischung wurde vorgelegt und eine wässrige Lösung des Matrixpolymers zugegeben. Anschließend wurden die übrigen Inhaltsstoffe eingerührt. Die Lösung wurde ausgestrichen und getrocknet, um einen oralen Dünnfilm zu erhalten.

Es ist zu erkennen, dass die Mischung aus (S)-Ketamin (freie Base) und (S)-Ketaminhydrochlorid (4) im Wesentlichen den gleichen pH-Wert aufweist, wie die neutralisierte Formulierung (2).

Vorteile der Formulierung (4), umfassend (S)-Ketamin (freie Base) und (S)-Ketaminhydrochlorid ohne Zugabe von NaOH sind:
Keine Salzbildung durch die Neutralisation und keine damit verbundenen negativen Effekte.

Durch das niedrigere Molekulargewicht von (S)-Ketamin (freie Base) ist der Anteil an eingesetztem Wirkstoff geringer, d.h. Anteile anderer Komponenten können relativ dazu erhöht werden.

(S)-Ketamin (freie Base) und die korrespondierende protonierte Verbindung bilden ein Puffersystem.

(S)-Ketamin (freie Base) wird durch das HCl zusätzlich stabilisiert.

Potenzielle Geschmacksverbesserung, da der pH-Wert einen Einfluss auf den Geschmack haben kann (sauer bzw. basisch (= seifig)).

Durch Variation der Menge an (S)-Ketamin (freie Base) und (S)-Ketaminhydrochlorid kann ein beliebiger pH-Wert zwischen dem der Base und dem Salz eingestellt werden.

## Patentansprüche

1. Oraler Dünnfilm, umfassend mindestens ein Matrixpolymer und mindestens einen pharmazeutisch aktiven Wirkstoff, wobei der mindestens eine pharmazeutisch aktive Wirkstoff eine Säure oder eine Base ist, **dadurch gekennzeichnet, dass** der mindestens eine pharmazeutisch aktive Wirkstoff in Form einer Mischung vorliegt, die den mindestens einen pharmazeutisch aktiven Wirkstoff sowohl in Form der freien Säure oder Base als auch in Form eines pharmazeutisch akzeptablen Salzes umfasst, wobei die Mischung, die den mindestens einen pharmazeutisch aktiven Wirkstoff sowohl in Form der freien Säure oder Base als auch in Form eines pharmazeutisch akzeptablen Salzes umfasst, den mindestens einen pharmazeutisch aktiven Wirkstoff in Form der freien Säure oder Base und den mindestens einen pharmazeutisch aktiven Wirkstoff in Form eines pharmazeutisch akzeptablen Salzes der freien Säure oder Base im molaren Verhältnis von 3:1 bis 1:3 umfasst.

2. Oraler Dünnfilm gemäß Anspruch 1, **dadurch gekennzeichnet, dass** das mindestens eine Matrixpolymer ein wasserlösliches Polymer umfasst.

3. Oraler Dünnfilm gemäß irgendeinem der vorhergehenden Ansprüche, **dadurch gekennzeichnet, dass** das mindestens eine Matrixpolymer ausgewählt ist aus der Gruppe umfassend Stärke und Stärkederivate, Dextrane, Cellulosederivate, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylate, Polyvinylpyrrolidone, Polyvinylalkohol-Polyethylenglycol-Pfropfcopolymere, Vinvlpyrrolidon/Vinylacetat-Copolymere, Polyvinylalkohole, Polyethylenoxidpolymere, Polyacrylamide, Polyethylenglycole, Gelatine, Collagen, Alginaten, Pektin, Pullulan, Traganth, Chitosan, Alginsäure, Arabinogalaktan, Galaktomannan, Agar, Agarose, Carrageen und natürlichen Gummen.

4. Oraler Dünnfilm gemäß irgendeinem der vorhergehenden Ansprüche, **dadurch gekennzeichnet, dass** das mindestens eine Matrixpolymer in einer Menge von 10 bis 90 Gew.-%, bezogen auf das Gesamtgewicht des oralen Dünnfilms, vorliegt.

5. Oraler Dünnfilm gemäß irgendeinem der vorhergehenden Ansprüche, **dadurch gekennzeichnet, dass** der orale Dünnfilm neben der Mischung, die den mindestens einen pharmazeutisch aktiven Wirkstoff sowohl in Form der freien Säure oder Base als auch in Form eines pharmazeutisch akzeptablen Salzes umfasst, keine weiteren Säure Basen, Salze und/oder Puffersysteme umfasst.

6. Oraler Dünnfilm gemäß irgendeinem der vorhergehenden Ansprüche, **dadurch gekennzeichnet, dass** der mindestens eine pharmazeutisch aktive Wirkstoff eine Carboxylgruppe, eine Aminogruppe, eine Sulfongruppe und/oder eine Phosphonatgruppe umfasst.

7. Oraler Dünnfilm gemäß irgendeinem der vorhergehenden Ansprüche, **dadurch gekennzeichnet, dass** der mindestens eine pharmazeutisch aktive Wirkstoff ausgewählt ist aus der Gruppe, umfassend die Wirkstoffklassen der Analgetika, Hormone, Hypnotika, Sedativa, Antiepileptika, Weckamine, Psychoneurotropika, Neuro-Muskelblocker, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormone, Sexualhormone, Antidiabetika, Antitumor-Wirkstoffe, Antibiotika, Chemotherapeutika und Narcotika.

8. Oraler Dünnfilm gemäß irgendeinem der vorhergehenden Ansprüche, **dadurch gekennzeichnet, dass** der mindestens eine pharmazeutisch aktive Wirkstoff Ketamin, besonders bevorzugt (S)-Ketamin, umfasst.

9. Oraler Dünnfilm gemäß irgendeinem der vorhergehenden Ansprüche, **dadurch gekennzeichnet, dass** die Mischung, die den mindestens einen pharmazeutisch aktiven Wirkstoff sowohl in Form der freien Säure oder Base als auch in Form eines pharmazeutisch akzeptablen Salzes umfasst, Ketamin als freie Base und Ketaminhydrochlorid, bevorzugt (S)-Ketamin als freie Base und (S)-Ketaminhydrochlorid, umfasst.

10. Oraler Dünnfilm gemäß irgendeinem der vorhergehenden Ansprüche, **dadurch gekennzeichnet, dass** die Mischung, die den mindestens einen pharmazeutisch aktiven Wirkstoff sowohl in Form der freien Säure oder Base als auch in Form eines pharmazeutisch akzeptablen Salzes umfasst, in einer Menge von 35 bis 55 Gew.-%, bezogen auf das Gesamtgewicht des oralen Dünnfilms, vorliegt.

11. Oraler Dünnfilm gemäß irgendeinem der vorhergehenden Ansprüche, **dadurch gekennzeichnet, dass** die Mischung, die den mindestens einen pharmazeutisch aktiven Wirkstoff sowohl in Form der freien Säure oder Base als auch in Form eines pharmazeutisch akzeptablen Salzes umfasst, den mindestens einen pharmazeutisch aktiven Wirkstoff in Form der freien Säure oder Base und den mindestens einen pharmazeutisch aktiven Wirkstoff in Form eines pharmazeutisch akzeptablen Salzes im molaren Verhältnis von 1,5:1 bis 1:1,5 umfasst.

12. Oraler Dünnfilm gemäß irgendeinem der vorhergehenden Ansprüche, **dadurch gekennzeichnet, dass** der orale Dünnfilm einen pH von 3,5 bis 9,5, vorzugsweise von 4,5 bis 8,8 aufweist.

13. Oraler Dünnfilm gemäß irgendeinem der vorhergehenden Ansprüche, **dadurch gekennzeichnet, dass** der orale Dünnfilm ferner mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, Feuchthaltemittel, Konservierungsmittel und/oder Antioxidationsmittel, umfasst.

14. Verfahren zur Herstellung eines oralen Dünnfilm gemäß irgendeinem der Ansprüche 1 bis 13, umfassend die Schritte:
a) Herstellen einer Lösung, Dispersion oder Schmelze, die mindestens das mindestens eine Matrixpolymer und den mindestens einen pharmazeutischen Wirkstoff in Form einer Mischung, die den mindestens einen pharmazeutisch aktiven Wirkstoff sowohl in Form der freien Säure oder Base als auch in Form eines pharmazeutisch akzeptablen Salzes umfasst, , wobei die Mischung, die den mindestens einen pharmazeutisch aktiven Wirkstoff sowohl in Form der freien Säure oder Base als auch in Form eines pharmazeutisch akzeptablen Salzes umfasst, den mindestens einen pharmazeutisch aktiven Wirkstoff in Form der freien Säure oder Base und den mindestens einen pharmazeutisch aktiven Wirkstoff in Form eines pharmazeutisch akzeptablen Salzes der freien Säure oder Base im molaren Verhältnis von 3:1 bis 1:3 umfasst, enthält,
b) Ausstreichen der Lösung, Dispersion oder Schmelze aus Schritt a), um einen Film zu erhalten, und
c) Trocknen des Films aus Schritt b), um einen oralen Dünnfilm zu erhalten.

15. Verwendung des oralen Dünnfilms gemäß irgendeinem der Ansprüche 1 bis 13 oder eines mehrschichtigen oralen Dünnfilms erhältlich gemäß dem Verfahren nach Anspruch 14 als Arzneimittel.

## Claims

1. An oral thin comprising at least one matrix polymer and at least one active pharmaceutical ingredient, wherein the at least one active pharmaceutical ingredient is an acid or a base, **characterised in that** the at least one active pharmaceutical ingredient is present in the form of a mixture which comprises the at least one active pharmaceutical ingredient both in the form of the free acid or base and in the form of a pharmaceutically acceptable salt, wherein the mixture comprising the at least one active pharmaceutical ingredient both in the form of the free acid or base and in the form of a pharmaceutically acceptable salt comprises the at least one active pharmaceutical ingredient in the form of the free acid or base and the at least one active pharmaceutical ingredient in the form of a pharmaceutically acceptable salt of the free acid or base in a molar ratio of 3:1 to 1:3.

2. The oral thin film according to claim 1, **characterised in that** the at least one matrix polymer comprises a water-soluble polymer.

3. The oral thin film according to any one of the preceding claims, **characterised in that** the at least one matrix polymer is selected from the group comprising starch and starch derivatives, dextrans, cellulose derivatives, such as carboxymethyl cellulose, hydroxypropyl cellulose, hydroxyethyl cellulose, hydroxypropyl methyl cellulose, hydroxypropyl ethyl cellulose, sodium carboxymethyl cellulose, ethyl or propyl cellulose, polyacrylic acids, polyacrylates, polyvinylpyrrolidones, polyvinyl alcohol-polyethylene glycol graft copolymers, vinylpyrrolidone/vinyl acetate copolymers, polyvinyl alcohols, polyethylene oxide polymers, polyacrylamides, polyethylene glycols, gelatines, collagen, alginates, pectin, pullulan, tragacanth, chitosan, alginic acid, arabinogalactan, galactomannan, agar, agarose, carrageenan, and natural gums.

4. The oral thin film according to any one of the preceding claims, **characterised in that** the at least one matrix polymer is present in an amount of 10 to 90 wt.%, in relation to the total weight of the oral thin film.

5. The oral thin film according to any one of the preceding claims, **characterised in that** the oral thin film, besides the mixture comprising the at least one active pharmaceutical ingredient both in the form of the free acid or base and in the form of a pharmaceutically acceptable salt, does not comprise any further acids, bases, salts and/or buffer systems.

6. The oral thin film according to any one of the preceding claims, **characterised in that** the at least one active pharmaceutical ingredient comprises a carboxyl group, an amino group, a sulfonyl group and/or a phosphonate group.

7. The oral thin film according to any one of the preceding claims, **characterised in that** the at least one active pharmaceutical ingredient is selected from the group comprising the active ingredient classes of analgesics, hormones, hypnotics, sedatives, antiepileptics, analeptics, psychoneurotropic drugs, neuro-muscle blockers, antispasmodics, antihistamines, antiallergics, cardiotonics, antiarrhythmics, diuretics, hypotensives, vasopressors, antidepressants, antitussives, expectorants, thyroid hormones, sexual hormones, antidiabetics, antitumour active ingredients, antibiotics, chemotherapeutics and narcotics.

8. The oral thin film according to any one of the preceding claims, **characterised in that** the at least one active pharmaceutical ingredient comprises ketamine, especially preferably (S)-ketamine.

9. The oral thin film according to any one of the preceding claims, **characterised in that** the mixture comprising the at least one active pharmaceutical ingredient both in the form of the free acid or base and in the form of a pharmaceutically acceptable salt comprises ketamine as free base and ketamine hydrochloride, preferably (S)-ketamine as free base and (S)-ketamine hydrochloride.

10. The oral thin film according to any one of the preceding claims, **characterised in that** the mixture comprising the at least one active pharmaceutical ingredient both in the form of the free acid or base and in the form of a pharmaceutically acceptable salt is present in an amount of 35 to 55 wt.%, in relation to the total weight of the oral thin film.

11. The oral thin film according to any one of the preceding claims, **characterised in that** the mixture comprising the at least one active pharmaceutical ingredient both in the form of the free acid or base and in the form of a pharmaceutically acceptable salt comprises the at least one active pharmaceutical ingredient in the form of the free acid or base and the at least active pharmaceutical ingredient in the form of a pharmaceutically acceptable salt in a molar ratio of 1.5:1 to 1:1.5.

12. The oral thin film according to any one of the preceding claims, **characterised in that** the oral thin film has a pH of 3.5 to 9.5, preferably of 4.5 to 8.8

13. The oral thin film according to any one of the preceding claims, **characterised in that** the oral thin film further comprises at least one auxiliary selected from the group comprising colouring agents, flavourings, sweeteners, plasticisers, taste-masking agents, emulsifiers, enhancers, humectants, preservatives and/or antioxidants.

14. A method for producing an oral thin film according to any one of claims 1 to 13, comprising the steps of:
a) producing a solution, dispersion or melt containing at least the at least one matrix polymer and the at least one active pharmaceutical ingredient in the form of a mixture comprising the at least one active pharmaceutical ingredient both in the form of the free acid or base and in the form of a pharmaceutically acceptable salt, wherein the mixture comprising the at least one active pharmaceutical ingredient both in the form of the free acid or base and in the form of a pharmaceutically acceptable salt contains the at least one active pharmaceutical ingredient in the form of the free acid or base and the at least one active pharmaceutical ingredient in the form of a pharmaceutically acceptable salt of the free acid or base in a molar ratio of 3:1 to 1:3.
b) spreading the solution, dispersion or melt from step a) in order to obtain a film, and
c) drying the film step b) in order to obtain an oral thin film.

15. Use of the oral thin film according to any one of claims 1 to 13 or of a multilayer oral thin film obtainable by the method according to claim 14 as a medicament.

## Revendications

1. Film mince oral comprenant au moins un polymère matriciel et au moins un principe pharmaceutiquement actif, dans lequel l'au moins un principe pharmaceutiquement actif est un acide ou une base, **caractérisé en ce que** l'au moins un principe pharmaceutiquement actif est présent sous forme d'un mélange, qui comprend l'au moins un principe pharmaceutiquement actif à la fois sous la forme de l'acide ou de la base libre et sous la forme d'un sel pharmaceutiquement acceptable, dans lequel le mélange, qui comprend l'au moins un principe pharmaceutiquement actif à la fois sous la forme de l'acide ou de la base libre et sous la forme d'un sel pharmaceutiquement acceptable, comprend l'au moins un principe pharmaceutiquement actif sous la forme de l'acide ou de la base libre et l'au moins un principe pharmaceutiquement actif sous la forme d'un sel pharmaceutiquement acceptable de l'acide ou de la base libre selon un rapport molaire de 3:1 à 1:3.

2. Film mince oral selon la revendication 1, **caractérisé en ce que** l'au moins un polymère matriciel comprend un polymère hydrosoluble.

3. Film mince oral selon l'une quelconque des revendications précédentes, **caractérisé en ce que** l'au moins un polymère matriciel est choisi parmi le groupe comprenant l'amidon et les dérivés d'amidon, les dextranes, les dérivés de cellulose, tels que la carboxyméthylcellulose, l'hydroxypropylcellulose, l'hydroxyéthylcellulose, l'hydroxypropylméthylcellulose, l'hydroxypropyléthylcellulose, la carboxyméthylcellulose de sodium, l'éthylcellulose ou la propylcellulose, les acides polyacryliques, les polyacrylates, les polyvinylpyrrolidones, les copolymères greffés d'alcool polyvinylique-de polyéthylène glycol, les copolymères de vinylpyrrolidone/acétate de vinyle, les alcools polyvinyliques, les polymères d'oxyde de polyéthylène, les polyacrylamides, les polyéthylènes glycol, la gélatine, le collagène, les alginates, la pectine, le pullulane, la gomme adragante, le chitosan, l'acide alginique, l'arabinogalactane, le galactomannane, la gélose, l'agarose, le carraghénane et les gommes naturelles.

4. Film mince oral selon l'une quelconque des revendications précédentes, **caractérisé en ce que** l'au moins un polymère matriciel est présent en une quantité de 10 à 90 % en poids, par rapport au poids total du film mince oral.

5. Film mince oral selon l'une quelconque des revendications précédentes, **caractérisé en ce que** le film mince oral ne comprend pas d'autres acides, bases, sels et/ou systèmes tampons en plus du mélange, qui comprend l'au moins un principe pharmaceutiquement actif à la fois sous la forme de l'acide ou de la base libre et sous la forme d'un sel pharmaceutiquement acceptable.

6. Film mince oral selon l'une quelconque des revendications précédentes, **caractérisé en ce que** l'au moins un principe pharmaceutiquement actif comprend un groupe carboxyle, un groupe amino, un groupe sulfonique et/ou un groupe phosphonate.

7. Film mince oral selon l'une quelconque des revendications précédentes, **caractérisé en ce que** l'au moins un principe pharmaceutiquement actif est choisi parmi le groupe comprenant les classes de principes actifs des analgésiques, des hormones, des hypnotiques, des sédatifs, des antiépileptiques, des weckamines, des psychoneurotropes, des bloqueurs neuromusculaires, des antispasmodiques, des antihistaminiques, des antiallergiques, des cardiotoniques, des antiarythmiques, des diurétiques, des hypotenseurs, des vasopresseurs, des antidépresseurs, des antitussifs, des expectorants, des hormones thyroïdiennes, des hormones sexuelles, des antidiabétiques, des principes actifs antitumoraux, des antibiotiques, des agents chimiothérapeutiques et des narcotiques.

8. Film mince oral selon l'une quelconque des revendications précédentes, **caractérisé en ce que** l'au moins un principe pharmaceutiquement actif comprend de la kétamine, de manière particulièrement préférée la (S)-kétamine.

9. Film mince oral selon l'une quelconque des revendications précédentes, **caractérisé en ce que** le mélange, qui comprend l'au moins un principe pharmaceutiquement actif à la fois sous la forme de l'acide ou de la base libre et sous la forme d'un sel pharmaceutiquement acceptable, comprend de la kétamine en tant que base libre et de l'hydrochlorure de kétamine, de manière préférée de la (S)-kétamine en tant que base libre et du (S)-hydrochlorure de kétamine.

10. Film mince oral selon l'une quelconque des revendications précédentes, **caractérisé en ce que** le mélange, qui comprend l'au moins un principe pharmaceutiquement actif à la fois sous la forme de l'acide ou de la base libre et sous la forme d'un sel pharmaceutiquement acceptable, est présent en une quantité de 35 à 55 % en poids, par rapport au poids total du film mince oral.

11. Film mince oral selon l'une quelconque des revendications précédentes, **caractérisé en ce que** le mélange, qui comprend l'au moins un principe pharmaceutiquement actif à la fois sous la forme de l'acide ou de la base libre et sous la forme d'un sel pharmaceutiquement acceptable, comprend l'au moins un principe pharmaceutiquement actif sous la forme de l'acide ou de la base libre et l'au moins un principe pharmaceutiquement actif sous la forme d'un sel pharmaceutiquement acceptable selon un rapport molaire de 1,5:1 à 1:1,5.

12. Film mince oral selon l'une quelconque des revendications précédentes, **caractérisé en ce que** le film mince oral présente un pH de 3,5 à 9,5, de préférence de 4,5 à 8,8.

13. Film mince oral selon l'une quelconque des revendications précédentes, **caractérisé en ce que** le film mince oral comprend en outre au moins un excipient choisi parmi le groupe comprenant les colorants, les arômes, les édulcorants, les plastifiants, les agents de masquage du goût, les émulsifiants, les exhausteurs, les humectants, les conservateurs et/ou les antioxydants.

14. Procédé de fabrication d'un film mince oral selon l'une quelconque des revendications 1 à 13, comprenant les étapes :
a) de fabrication d'une solution, d'une dispersion ou d'une matière fondue, qui comprend au moins l'au moins un polymère matriciel et l'au moins un principe pharmaceutiquement actif sous la forme d'un mélange, qui comprend l'au moins un principe pharmaceutiquement actif à la fois sous la forme de l'acide ou de la base libre et sous la forme d'un sel pharmaceutiquement acceptable, dans lequel le mélange, qui comprend l'au moins un principe pharmaceutiquement actif à la fois sous la forme de l'acide ou de la base libre et sous la forme d'un sel pharmaceutiquement acceptable, contient l'au moins un principe pharmaceutiquement actif sous forme de l'acide ou de la base libre et l'au moins un principe pharmaceutiquement actif sous la forme d'un sel pharmaceutiquement acceptable de l'acide ou de la base libre selon un rapport molaire de 3:1 à 1:3 ;
b) l'étalement de la solution, de la dispersion ou de la matière fondue de l'étape a) pour obtenir un film ; et
c) le séchage du film de l'étape b) pour obtenir un film mince oral.

15. Utilisation du film mince oral selon l'une quelconque des revendications 1 à 13 ou d'un film mince oral multicouche pouvant être obtenu selon le procédé selon la revendication 14 en tant que médicament.
